(19) Europäisches Patentamt / European Patent Office / Office européen des brevets

(11) **EP 1 627 661 B1**

(12) **FASCICULE DE BREVET EUROPEEN**

(45) Date de publication et mention
de la délivrance du brevet:
**23.12.2009 Bulletin 2009/52**

(51) Int Cl.:
***A61N 1/365*** *(2006.01)*

(21) Numéro de dépôt: **05291745.7**

(22) Date de dépôt: **18.08.2005**

(54) **Dispositif médical implantable actif comprenant des moyens d'évaluation du volume intracardiaque**

Implantierbare medizinische aktive Vorrichtung mit Bewertung des intrakardialen Volumen

Implantable medical active system with evaluating of intracardiale volume

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **18.08.2004 FR 0408955**

(43) Date de publication de la demande:
**22.02.2006 Bulletin 2006/08**

(73) Titulaire: **ELA MEDICAL**
**92541 Montrouge (FR)**

(72) Inventeur: **Cooper, Daniel**
**38330 Saint Ismier (FR)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**US-A1- 2001 034 540     US-A1- 2004 127 944**
**US-A1- 2004 147 968**

**Description**

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne en particulier les dispositifs comprenant des moyens de mesure d'une bioimpédance intracardiaque, typiquement de mesure d'une bioimpédance transvalvulaire, transseptale ou intraventriculaire, techniques en elles-mêmes connues.

La bioimpédance intracardiaque est un paramètre étroitement corrélé au débit cardiaque et donc à la fraction d'éjection, paramètres qui peuvent être utiles notamment pour piloter certains paramètres de stimulation tels que la fréquence cardiaque et/ou le délai atrioventriculaire dans un sens permettant de maximiser le débit, ou encore pour piloter le délai interventriculaire dans le cas d'une stimulation biventriculaire (resynchronisation des ventricules).

Le EP-A-1116497 (ELA Medical) décrit ainsi une mesure dynamique de bioimpédance intracardiaque transvalvulaire entre oreillette et ventricule situés d'un même côté du coeur, et le EP-A-1138346 (ELA Medical) décrit une mesure de bioimpédance transseptale, c'est-à-dire entre un site situé d'un côté du coeur et un site situé de l'autre côté du coeur, la configuration pouvant être transseptale oblique (entre un ventricule et l'oreillette situés du côté opposé) ou transseptale interventriculaire (entre les deux ventricules).

Concrètement, la bioimpédance intracardiaque est mesurée par injection d'un courant et recueil d'un potentiel différentiel à des pôles respectifs, différents, d'une configuration tripolaire ou quadripolaire d'électrodes placées à l'intérieur du myocarde (électrode auriculaire, électrode ventriculaire proximale, électrode ventriculaire distale, etc.).

La bioimpédance, quelle que soit la façon dont elle est mesurée, est un paramètre dynamique (c'est-à-dire variant de façon continue au cours d'un même cycle cardiaque) donnant une indication du débit cardiaque instantané. Plus précisément, les variations de la bioimpédance intracardiaque dépendent principalement de celles du volume des cavités cardiaques, une impédance faible correspondant à un volume élevé, et réciproquement. L'impédance varie donc entre un minimum, atteint à la fin de la phase diastolique, et un maximum, atteint à la fin de la phase systolique. La différence entre l'impédance systolique et l'impédance diastolique donne une valeur corrélée au volume d'éjection, valeur à partir de laquelle on peut évaluer le débit cardiaque, qui est le produit du volume d'éjection par la fréquence cardiaque.

Le US 2004/0127944- EP 1 405 654 (ELA Medical) décrit une telle technique de calcul du volume d'éjection, permettant d'estimer périodiquement sur une pluralité de cycles un indice représentatif de l'évolution du débit cardiaque, sur lequel document s'appuie le préambule de la revendication 1.

Les indications fournies par ces mesures sont cependant des indications relatives des volumes systolique et diastolique, c'est-à-dire que l'on détermine la différence (volume d'éjection) entre ces volumes, ce qui, avec la connaissance du rythme, est suffisant si l'on souhaite seulement évaluer le débit cardiaque. En revanche, ces dispositifs ne donnent pas accès à une mesure absolue du volume intracardiaque.

Le point de départ de l'invention est la constatation que, dans certaines circonstances, il serait intéressant de disposer d'un signal représentatif non plus, ou plus seulement, du volume d'éjection, mais de la valeur absolue du volume intracardiaque, notamment à des fins diagnostiques.

Jusqu'à présent, la valeur absolue du volume intracardiaque est estimée à partir de signaux échographiques, qui permet d'évaluer le volume diastolique et le volume systolique, c'est-à-dire les deux valeurs extrêmes du volume intracardiaque, et donne une indication sur la manière dont varie le volume entre ces deux extrêmes au cours d'un même cycle. Mais :

- bien évidemment, cette technique n'est applicable que lorsque le patient subit un examen, donc d'une manière ponctuelle qui ne permet pas un suivi permanent, sur le long terme,
- les valeurs obtenues sont des valeurs calculées, qui sont affichées ou imprimées par l'appareil échographique mais ne constituent pas des signaux qui pourraient par exemple permettre le pilotage d'une fonction d'un stimulateur ou le diagnostic de certaines pathologies par analyse de l'évolution de ce signal. L'invention a pour objet de proposer un dispositif médical implantable actif permettant d'évaluer la valeur absolue, instantanée, du volume intracardiaque, pour remédier aux inconvénients ci-dessus et accroître les possibilités de pilotage et de diagnostic automatique des dispositifs implantés.

A cet effet, l'invention propose un dispositif du type décrit notamment dans les EP-A-1116497 et EP-A-1138346 précités, c'est-à-dire comprenant : une pluralité de bornes de liaison aptes à être reliées à des électrodes placées en au moins trois sites respectifs distincts d'un myocarde, et des moyens de mesure d'une bioimpédance intracardiaque, comprenant des moyens d'injection d'un courant et de recueil d'un potentiel différentiel à des pôles respectifs d'une configuration desdites bornes, et des moyens aptes à délivrer en sortie un signal dynamique d'impédance fonction du courant injecté et du potentiel différentiel correspondant recueilli.

De façon caractéristique de l'invention, le dispositif comprend en outre des moyens évaluateurs de volume intracardiaque, recevant en entrée le signal d'impédance et délivrant en sortie une valeur dynamique de volume, représentative de la valeur absolue instantanée d'un volume intracardiaque.

Dans une première forme de mise en oeuvre de l'invention, les moyens évaluateurs sont des moyens aptes à opérer la conversion du signal d'impédance en volume selon un caractéristique linéaire de la forme Vol = $a.Z + b$, Vol étant la valeur de volume délivrée par les moyens évaluateurs, Z étant la valeur du signal d'impédance reçue en entrée et $a$ et $b$ étant des constantes.

Dans une seconde forme, préférentielle, de mise en oeuvre de l'invention, les moyens évaluateurs sont des moyens aptes à opérer la conversion du signal d'impédance en volume selon un caractéristique hyperbolique de la forme Vol = $a/(Z + b)$, Vol étant la valeur de volume délivrée par les moyens évaluateurs, Z étant la valeur du signal d'impédance reçue en entrée et $a$ et $b$ étant des constantes.

Dans l'un ou l'autre cas, des moyens de calibrage peuvent déterminer préalablement lesdites constantes a et b à partir d'au moins deux couples de valeurs de volume relevées et de valeurs d'impédance mesurées correspondantes.

On va maintenant décrire un exemple de réalisation de l'invention, en référence aux dessins annexés.

La figure 1 illustre de façon schématique un exemple de technique de mesure d'une bioimpédance intracardiaque.

La figure 2 est un diagramme impédance/volume montrant les résultats obtenus avec la technique de l'invention, respectivement pour une conversion linéaire et pour une conversion hyperbolique, par rapport à des valeurs effectivement mesurées dans le cas d'une essai clinique réel.

La figure 3 est une caractéristique du même type que celle de la figure 2, montrant en outre, à titre de comparaison, les résultats d'une simulation numérique par la méthode des éléments finis.

On va maintenant décrire un exemple de réalisation du dispositif de l'invention, qui peut être mise en oeuvre par une programmation appropriée du logiciel de commande d'un appareil tel que les dispositifs implantés commercialisés par ELA Médical, Montrouge, France. Il s'agit de dispositifs programmables à microprocesseur comportant des circuits pour recevoir, mettre en forme et traiter des signaux électriques recueillis par des électrodes implantées. Il est possible d'y transmettre par télémétrie des logiciels qui seront implantés en mémoire et exécutés pour mettre en oeuvre les fonctions de l'invention décrites ci-dessous. L'adaptation de ces appareils à la mise en oeuvre des fonctions de l'invention est à la portée de l'homme du métier, et elle ne sera pas décrite en détail.

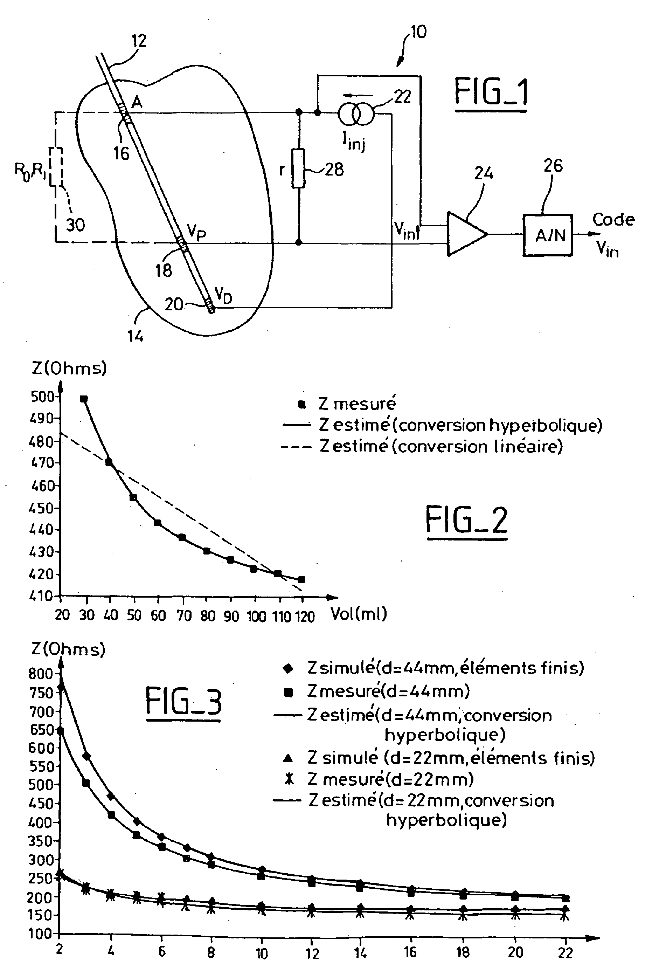

Sur la figure 1, la référence 10 désigne, de façon générale, un circuit de mesure de bioimpédance intracardiaque, en pratique incorporé au circuit du générateur d'un stimulateur implanté.

La référence 12 désigne une sonde endocavitaire implantée dans le myocarde 14 et comportant, dans l'exemple illustré, une électrode auriculaire 16, une électrode ventriculaire proximale 18 et une électrode ventriculaire distale 20.

Pour mesure l'impédance intracardiaque (dans cet exemple une impédance transvalvulaire), le dispositif injecte au moyen d'un générateur 22 un courant $I_{inj}$ entre l'électrode auriculaire 16 et l'électrode ventriculaire distale 20. Un potentiel différentiel correspondant Vin est alors recueilli entre l'électrode auriculaire 16 (qui est donc une électrode commune à l'injection et au recueil) et l'électrode ventriculaire proximale 18. Cette tension est amplifiée par un amplificateur 24 et numérisée par un convertisseur analogique/numérique 26, pour donner une valeur codée représentative de la tension $V_{in}$.

Cette technique de mesure est en elle-même connue et ne sera pas décrite plus en détail. On notera seulement qu'il ne s'agit là que d'un exemple de configuration de mesure de bioimpédance intracardiaque, sans caractère limitatif.

La première étape (en elle-même connue) du traitement des signaux consiste à évaluer l'impédance à partir des valeurs du courant injecté Iinj et de la tension différentielle $V_{in}$ recueille.

Cette impédance est donnée par la relation :

$$Z = K_1 . \frac{(V_{in} - K_3)}{I_{inj}} - K_2 ,$$

$K_1$ étant le facteur de proportionnalité entre la tension d'entrée et un pas du convertisseur 26 (exprimé en $\mu$V par pas de code), $K_2$ étant l'impédance interne de l'implant, illustrée symboliquement en 28 par la résistance r (exprimée en ohms) et $K_3$ étant la valeur de décalage, c'est-à-dire la valeur délivrée par le convertisseur 26 pour une tension d'entrée $V_{in}$ nulle (exprimée en pas de code).

Pour déterminer les trois constantes $K_1$, $K_2$ et $K_3$, il est nécessaire d'opérer préalablement trois mesures avec des valeurs différentes $I_{inj1}$ et $I_{inj2}$ du courant d'injection et des valeurs différentes de l'impédance entre les bornes. Ce calibrage peut être effectué par exemple en substituant à la sonde 12 une résistance 30 de valeur fixe $R_0$ ou $R_1$ connue, l'une des deux valeurs pouvant être par exemple $R_0 = 0$ (mise en court-circuit de l'entrée).

Le calibrage peut être effectué pour chaque implant, lors d'un test automatisé sur chaîne de fabrication, les valeurs spécifiques des constantes $K_1$, $K_2$ et $K_3$ étant mémorisées dans l'implant après avoir été déterminées, $K_1$ étant la dérive de la tension d'entrée pour 1 pas du convertisseur, exprimée en $\mu$V par pas de code :

$$K_1 = I_{inj2} * R_1 / (V_{in} (R_1, I_{nj2}) - V_{in} (R_0, I_{inj2})),$$

$K_2$ étant l'impédance interne de l'implant, exprimée en ohms :

$$K_2 = (V_{in} (R_0, I_{inj2}) - V_{in} (R_0, I_{inj1}))* K_1 / (I_{inj2} - I_{inj1}),$$

et $K_3$ étant la valeur de sortie du convertisseur pour une tension d'entrée nulle (exprimée en pas de code) :

$$K_3 = V_{in} (R_1, I_{inj2}) - (R_1 + K_2) * I_{inj2} / K_1.$$

En variante, il est possible d'effectuer un calibrage seulement pour quelques échantillons représentatifs d'implants, déterminer une valeur moyenne pour les constantes $K_1$, $K_2$ et $K_3$, et appliquer ces constantes à tous les implants ; cette dernière manière de procéder est moins précise, mais se révèle en pratique suffisante pour la majorité des besoins courants, à l'exception de quelques protocoles de recherche nécessitant une précision accrue.
L'étape suivante, caractéristique de l'invention, consiste à déterminer à partir du signal d'impédance ainsi obtenu la valeur absolue, dynamique, du volume endocavitaire.
Dans une première forme de mise en oeuvre, le volume (Vol) est déterminé à partir d'une caractéristique volume/impédance qui est linéaire, c'est-à-dire une relation de la forme générale Vol = a.Z + b. Pour déterminer les deux constantes a et b, un calibrage est effectué à partir de deux points initiaux, connus par ailleurs, déterminés pour chaque patient, par exemple le volume systolique $Vol_1$ et le volume diastolique $Vol_2$, déterminés par échographie. A ces deux valeurs de volume $Vol_1$ et $Vol_2$ correspondent des valeurs d'impédance $Z_1$ et $Z_2$, d'où la relation suivante :

$$Vol = Vol_1 + \frac{(Z - Z_1).(Vol_1 - Vol_2)}{Z_1 - Z_2} .$$

Dans une seconde forme de mise en oeuvre, préférentielle, le volume est déterminé à partir d'une caractéristique volume/impédance qui est hyperbolique, c'est-à-dire une relation de la forme générale Vol = a/(Z + b) ou, en d'autres termes :

$$Vol = \frac{K_4}{Z - K_5}$$

Les deux constantes $K_4$ et $K_5$ sont, ici encore, déterminées à partir de mesures initiales obtenues pour chaque patient,
$K_4$ étant la constante de proportionnalité du volume, exprimée en millilitres par ohm :

$$K_4 = (Z_1 - Z_2) * Vol_1 / (1 - (Vol_1 / Vol_2))$$

et $K_5$ représentant le niveau de l'asymptote pour un volume tendant vers l'infini, exprimé en ohms :

$$K_5 = Z_1 - K_4 / Vol_1.$$

La figure 2 montre les résultats d'une estimation du volume effectuée de la manière indiquée ci-dessus, soit par une conversion linéaire (caractéristique en tiretés), soit par une conversion hyperbolique (caractéristique en trait plein). Les carrés représentent des points de mesure d'un essai réalisé *in vitro* sur un coeur de chien, documenté dans l'article de Raul Chirife *"Intracardiac Impedance for Hemodynamic Assessment"* publié dans le cadre du congrès *Cardiac Pacing,* Rome, 2004.

Comme on peut le voir, la valeur du volume estimée en utilisant une conversion hyperbolique est extrêmement proche de la réalité physique, quoiqu'une conversion linéaire, plus simple à mettre en oeuvre, puisse donner également des résultats satisfaisants lorsqu'une précision moindre est requise, ou sur une plage de variation de volume plus faible.

La figure 3 illustre, comme pour la figure 2, les résultats d'une estimation du volume effectuée de la manière indiquée ci-dessus, par une conversion hyperbolique, pour deux valeurs d = 22 mm et d = 44 mm du diamètre d'une chambre de test en silicone remplie de solution saline, par rapport à :

- d'une part des valeurs effectivement mesurées *in vitro* (carrés et étoiles) et,
- d'autre part des valeurs issues d'une simulation numérique par la méthode des éléments finis (losanges et triangles).

Les valeurs comparatives mesurées et estimées sont documentées dans l'article de K. Hoekstein et G.F. Inbar "Cardiac Stroke Volume Estimation", EE Pub. No. 974, Février 1994, Technion, Israël.

Ici encore, l'analyse de cette figure montre la pertinence d'une conversion volume/impédance réalisée selon les enseignements de l'invention, particulièrement d'une conversion de type hyperbolique.

## Revendications

1. Un dispositif médical implantable actif, notamment un stimulateur cardiaque, défibrillateur/cardioverteur et/ou dispositif multisite, comprenant :

   - une pluralité de bornes de liaison aptes à être reliées à des électrodes (16, 18, 20) placées en au moins trois sites respectifs distincts d'un myocarde (14), et
   - des moyens de mesure d'une bioimpédance intracardiaque, comprenant des moyens (22, 24) d'injection d'un courant ($I_{inj}$) et de recueil d'un potentiel différentiel ($V_{in}$) à des pôles respectifs d'une configuration desdites bornes, et des moyens aptes à délivrer en sortie un signal dynamique d'impédance (Z) fonction du courant injecté et du potentiel différentiel correspondant recueilli,

   **caractérisé en ce qu'**il comprend en outre :

   - des moyens évaluateurs de volume intracardiaque, recevant en entrée le signal d'impédance (Z) et délivrant en sortie une valeur dynamique de volume (Vol), représentative de la valeur absolue instantanée d'un volume intracardiaque.

2. Le dispositif de la revendication 1, dans lequel les moyens évaluateurs sont des moyens aptes à opérer la conversion du signal d'impédance en volume selon un caractéristique linéaire de la forme Vol = *a.Z + b*, Vol étant la valeur de volume délivrée par les moyens évaluateurs, Z étant la valeur du signal d'impédance reçue en entrée et a et b étant des constantes.

3. Le dispositif de la revendication 1, dans lequel les moyens évaluateurs sont des moyens aptes à opérer la conversion du signal d'impédance en volume selon un caractéristique hyperbolique de la forme Vol = *a*/(*Z* + b)*, Vol étant la valeur de volume délivrée par les moyens évaluateurs, Z étant la valeur du signal d'impédance reçue en entrée et a et b étant des constantes.

4. Le dispositif de la revendication 2 ou 3, comprenant en outre des moyens de calibrage, aptes à déterminer préalablement lesdites constantes a et b à partir d'au moins deux couples de valeurs de volume ($Vol_1$ ; $Vol_2$) relevées et de valeurs d'impédance ($Z_1$ ; $Z_2$) mesurées correspondantes.

## Claims

1. An active medical device, especially a cardiac pacemaker, a defibrillator/cardioverter and or a multisite device, comprising

- a plurality of terminals adapted to be connected to electrodes (16, 18, 20) situated on at least three respective distinct sites of a myocardium (14),
- means for measuring an intracardiac bioimpedance, comprising means (22, 24) for injecting a current ($I_{inj}$) and for acquiring a differential potential ($V_{in}$) on respective poles of a configuration of said terminals, and means adapted to deliver at the output an impedance dynamic signal (5) as a function of the injected current and of the acquired corresponding differential potential,

**characterized in that** it furthermore comprises

- means for evaluating the intracardiac volume, that receive at the input the impedance signal (Z) and deliver at the output a volume dynamic value (Vol) representative of the instantaneous absolute value of an intracardiac volume.

2. The device according to claim 1, wherein the evaluating means are means adapted to operate the conversion of the impedance signal into a volume according to a linear characteristic of the form Vol = $a.Z + b$, Z is the value of the impedance signal received at the input, *and a* and *b* are constants.

3. The device according to claim 1, wherein the evaluating means are means adapted to operate the conversion of the impedance signal into a volume according to a hyperbolic characteristic of the form Vol = $a/(Z + b)$ , Z is the value of the impedance signal received at the input, *and a* and *b* are constants.

4. The device according claim 2 or 3, furthermore comprising calibration means adapted to preliminarily determine said constants *a* und *b* based on at least two couples of picked up volume values ($Vol_1$ ; $Vol_2$) and of corresponding measured impedance values ($Z_1$ ; $Z_2$)

**Patentansprüche**

1. Ein aktives implantierbares medizinisches Gerät, insbesondere ein Herzschrittmacher, ein Defibrillator/ Kardioverter oder ein Multisite-Gerät, umfassend :

- eine Vielzahl von Anschlussklemmen, die geeignet sind, mit auf wenigstens drei respektiven, voneinander verschiedenen Stellen eines Myokards (14) befindlichen Elektroden (16, 18, 20) verbunden zu sein,
- Mittel zur Messung einer intrakardialen Bioimpedanz, umfassend Mittel (22, 24) zur Injektion eines Stroms ($I_{inj}$) und zum Auffangen eines Differenzialpotentials ($V_{in}$) auf respektiven Polen einer Konfiguration genannter Klemmen und Mittel, die geeignet sind, am Ausgang ein dynamisches Impedanzsignal (Z) in Abhängigkeit vom injektierten Strom und dem entsprechenden aufgefangenen Differenzialpotential zu liefern,

**dadurch gekennzeichnet, dass** es ausserdem

- Mittel zur Abschätzung des intrakardialen Volumens, die am Eingang das Impedanzsignal (Z) empfangen und am Ausgang einen dynamischen, für den instantanen Absolutwert repräsentativen Volumenwert (Vol) eines intrakardialen Volumens liefern,

umfasst.

2. Das Gerät gemäss Anspruch 1, in welchem die Abschätzungsmittel Mittel sind, die geeignet sind, die Konversion des Impedanzsignals in ein Volumen gemäss einer linearen Charakteristik der Form Vol = $a.Z + b$ durchzuführen, wobei Vol der Wert des durch die Abschätzungsmittel gelieferten Volumens, *Z* der Wert des am Eingang empfangenen Impedanzsignals und *a* und *b* Konstanten sind.

3. Das Gerät gemäss Anspruch 1, in welchem die Abschätzungsmittel Mittel sind, die geeignet sind, die Konversion des Impedanzsignals in ein Volumen gemäss einer hyperbolischen Charakteristik der Form Vol = $a/(Z + b)$ durchzuführen, wobei Vol der Wert des durch die Abschätzungsmittel gelieferten Volumens, *Z* der Wert des am Eingang empfangenen Impedanzsignals und *a* und *b* Konstanten sind.

4. Das Gerät gemäss Ansprüche 2 oder 3, ausserdem umfassend Kalibrierungsmittel, die geeignet sind, genannte Konstanten *a* und *b* basierend auf wenigstens zwei Paaren von aufgenommenen Volumenwerten ($Vol_1$ ; $Vol_2$) und

von entsprechenden gemessenen Impedanzwerten ($Z_1$ ; $Z_2$) vorläufig zu bestimmen.

FIG_1

FIG_2

- ■ Z mesuré
- —— Z estimé (conversion hyperbolique)
- - - - Z estimé (conversion linéaire)

FIG_3

- ◆ Z simulé (d=44mm, éléments finis)
- ■ Z mesuré (d=44mm)
- —— Z estimé (d=44mm, conversion hyperbolique)
- ▲ Z simulé (d=22mm, éléments finis)
- ✕ Z mesuré (d=22mm)
- —— Z estimé (d=22mm, conversion hyperbolique)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1116497 A
- EP 1138346 A
- US 20040127944 A
- EP 1405654 A

**Littérature non-brevet citée dans la description**

- **K. Hoekstein ; G.F. Inbar.** *''Cardiac Stroke Volume Estimation'',* Février 1994, vol. 974